# FASCICULE DE BREVET EUROPEEN

(11) **EP 2 136 849 B1**
(45) Date de publication et mention de la délivrance du brevet: **09.01.2013**
(21) Numéro de dépôt: 08787969.8
(22) Date de dépôt: 16.04.2008
(51) Int. Cl.: A61L 2/26, A61B 19/00, A61L 2/24

(54) **APPAREIL D'IMAGERIE MEDICAL AVEC UNE ENCEINTE DE DÉCONTAMINATION ET D'IDENTIFICATION D'UN INSTRUMENT MÉDICAL**
MEDIZINISCHE BILDGEBUNGSVORRICHTUNG MIT EINEM GEHÄUSE ZUR DEKONTAMINATION UND IDENTIFIKATION EINES MEDIZINISCHEN GERÄTES
MEDICAL IMAGING APPARATUS COMPRISING AN ENCLOSURE FOR THE DECONTAMINATION AND IDENTIFICATION OF A MEDICAL INSTRUMENT

(30) Priorité: 18.04.2007 FR 0754548
(43) Date de publication de la demande: 30.12.2009
(73) Titulaire: Germitec, 75008 Paris (FR)
(72) Inventeur: DESHAYS, Clément, F-07120 Ruoms (FR)
(74) Mandataire: Jacobson, Claude
(86) Numéro de dépôt international: PCT/FR2008/000541
(87) Numéro de publication internationale: WO 2008/142300

(56) Documents cités:
- EP-A- 0 839 537
- WO-A-2005/048041
- DE-A1- 19 917 206
- FR-A- 2 890 566
- US-A- 4 772 795

## Description

L'invention concerne un appareil d'imagerie médical comprenant au moins un instrument médical et une enceinte du type comportant une base, au moins une paroi latérale et un capot supérieur délimitant un volume de décontamination, une desdites au moins une paroi latérale comportant une ouverture d'accès au volume de décontamination s'étendant jusqu'au capot supérieur, le capot supérieur comportant un passage de câble ouvert vers ladite ouverture et un organe de suspension de câble au travers dudit passage de câble.

Les enceintes de ce type sont connues et utilisées par exemple pour la désinfection de la partie active d'une sonde médicale destinée à fonctionner avec un appareil d'imagerie.

Une telle enceinte est connue par example par le document FR-A-2 890 566.

La désinfection est par exemple effectuée par émission d'un rayonnement UV, par pulvérisation d'un liquide de désinfection sous forme de microgouttelettes ou par injection d'un gaz de désinfection dans le volume de décontamination de l'enceinte.

Dans une telle enceinte, la partie active de la sonde doit être suspendue dans le volume de décontamination afin de bénéficier de la plus grande surface de désinfection possible. En outre, il est préférable que le câble reliant la partie active de la sonde à l'appareil avec lequel la sonde est utilisée ne soit pas disposé intégralement dans l'enceinte afin de protéger la partie de connectique à l'appareil d'imagerie de ce câble et pour éventuellement permettre de ne pas débrancher la sonde de l'appareil d'imagerie lorsqu'elle est désinfectée. Enfin, il est primordial d'assurer l'étanchéité du volume de décontamination afin d'éviter toute fuite du principe actif de désinfection vers l'extérieur de l'enceinte.

Pour assurer une traçabilité de la désinfection d'une sonde afin d'éviter tout usage illégitime d'une sonde du point de vue hygiénique, il est important de pouvoir identifier sans erreur la sonde qui est désinfectée dans l'enceinte. Pour assurer une telle identification, l'utilisation d'étiquettes électroniques d'identification du type RFID est connue.

Cependant, dans un environnement où de nombreuses sondes sont présentes, par exemple si l'enceinte est rattachée à un appareil d'imagerie et que plusieurs sondes sont branchées sur cet appareil, il est possible qu'un lecteur RFID n'identifie pas la sonde en cours de désinfection, mais une sonde disposée à proximité, ce qui peut entraîner des erreurs graves dans la traçabilité de la désinfection d'une sonde.

Un des buts de l'invention est de proposer un appareil d'imagerie médical comprenent au moins un instrument médical et une enceinte de décontamination permettant d'assurer l'identification de la sonde dans l'enceinte de façon sûre.

A cet effet, l'invention prévoit une enceinte de décontamination du type précité, dans laquelle l'enceinte comprend des moyens de lecture d'une étiquette d'identification électronique prévue sur un câble de l'instrument médical, ladite étiquette étant disposée à proximité de la partie active à désinfecter de l'instrument médical, lesdits moyens de lecture étant agencés pour être à proximité de l'étiquette d'identification lorsque le câble est suspendu à l'organe de suspension de câble.

L'enceinte selon l'invention permet donc d'assurer que c'est bien la sonde présente dans l'enceinte qui est identifiée.

Selon d'autres caractéristiques de l'appareil d'imagerie médical de l'invention :
- les moyens de lecture d'une étiquette d'identification électronique comprennent un lecteur RFID de faible portée ;
- le passage de câble comporte une fente aménagée dans le capot supérieur ;
- le capot comporte un volet d'obturation mobile entre une position de fermeture du passage de câble, dans laquelle le volet ferme le passage de câble de manière hermétique, et une position d'ouverture du passage de câble ;
- l'enceinte comprend une porte de fermeture de l'ouverture d'accès au volume de décontamination, ladite porte étant agencée pour que sa fermeture entraîne le volet mobile vers sa position de fermeture du passage de câble ;
- l'organe de suspension comprend des moyens d'orientation du câble agencés pour disposer l'étiquette d'identification en regard des moyens de lecture lorsque le câble est suspendu à l'organe de suspension du câble ;
- l'organe de suspension de câble comporte un tube ouvert apte à s'emboîter de manière hermétique sur une portion de câble de l'instrument médical dont le diamètre est dans une plage de diamètres prédéterminés ; et
- l'organe de suspension comporte une pièce de réception d'une pièce de suspension prévue sur le câble de l'instrument médical, ladite pièce de réception et ladite pièce de suspension formant un ensemble d'assemblage constitué d'une pièce mâle et d'une pièce femelle.

La pièce de suspension de l'ensemble d'assemblage fixée à la portion de câble de l'instrument médical comporte une étiquette d'identification électronique.

D'autres aspects et avantages de l'invention apparaîtront au cours de la description qui suit, faite à titre d'exemple et en référence aux figures annexées.
La Fig. 1 est une représentation schématique en perspective d'un appareil d'imagerie médical auquel est fixée une enceinte de décontamination selon l'invention, une sonde étant disposée dans l'enceinte.
La Fig. 2 est une représentation schématique en perspective de l'enceinte de la Fig. 1 dans laquelle une sonde est disposée.
La Fig. 3 est une représentation schématique en perspective de l'appareil d'imagerie médical de la Fig. 1, l'enceinte étant ouverte.
La Fig. 4 est une représentation schématique en perspective de l'enceinte ouverte de la Fig. 3.
La Fig. 5 est une représentation schématique en coupe de l'ensemble d'assemblage du câble de la sonde et de l'enceinte, en cours d'assemblage.
La Fig. 6 est une représentation schématique en coupe selon la ligne VI-VI de la Fig. 2.

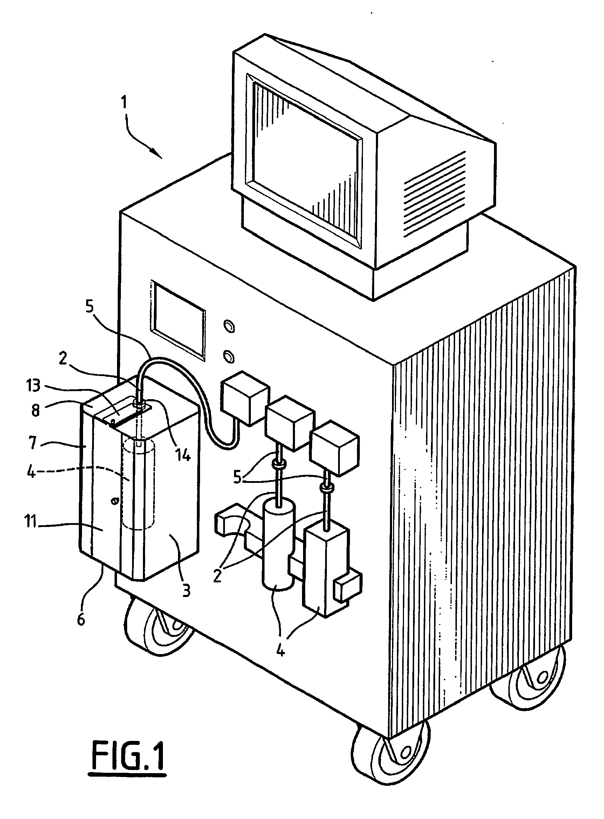

En référence à la Fig. 1, on décrit un appareil d'imagerie médical 1 susceptible de fonctionner avec une pluralité de sondes 2 et auquel est fixée une enceinte de décontamination 3 de la partie active 4 des sondes 2.

Dans le mode de réalisation représenté sur les figures, trois sondes 2 sont connectées à l'appareil d'imagerie 1. Selon d'autres modes de réalisation, un nombre différent de sondes peut être prévu. L'appareil d'imagerie 1 est connu en lui-même et ne sera pas décrit plus en détail ici. Une sonde 2 comprend une partie active 4 qui doit être désinfectée avant chaque utilisation et un câble 5 de connexion à l'appareil médical 1 et dont la partie de connectique ne doit pas être exposée au principe actif utilisé au cours de la désinfection. La sonde 2 est connue en elle-même et ne sera pas décrite plus en détail ici.

L'enceinte 3 comprend une base 6 formant le fond de l'enceinte, au moins une paroi latérale 7 formant le corps de l'enceinte et un capot supérieur 8. La paroi latérale 7 s'étend entre la base 6 et le capot supérieur de sorte à délimiter un volume de décontamination 9 dans lequel la partie active 4 de la sonde 2 est disposée afin d'être désinfectée.

Selon le mode de réalisation représenté sur les figures, l'enceinte 3 est sensiblement parallélépipédique et comprend quatre parois latérales 7. Selon d'autres modes de réalisation, l'enceinte 3 peut être sensiblement cylindrique de section circulaire ou autre.

L'enceinte 3 comprend des moyens (non représentés) de diffusion d'un principe actif de désinfection dans le volume de décontamination 9. Ces moyens sont par exemple une source d'émission d'UV, une buse de pulvérisation de microgouttelettes d'un liquide de désinfection ou une buse d'injection d'un gaz de désinfection. Les moyens de diffusion sont agencés pour que la partie active 4 de la sonde 2 soit uniformément exposée au principe actif de désinfection.

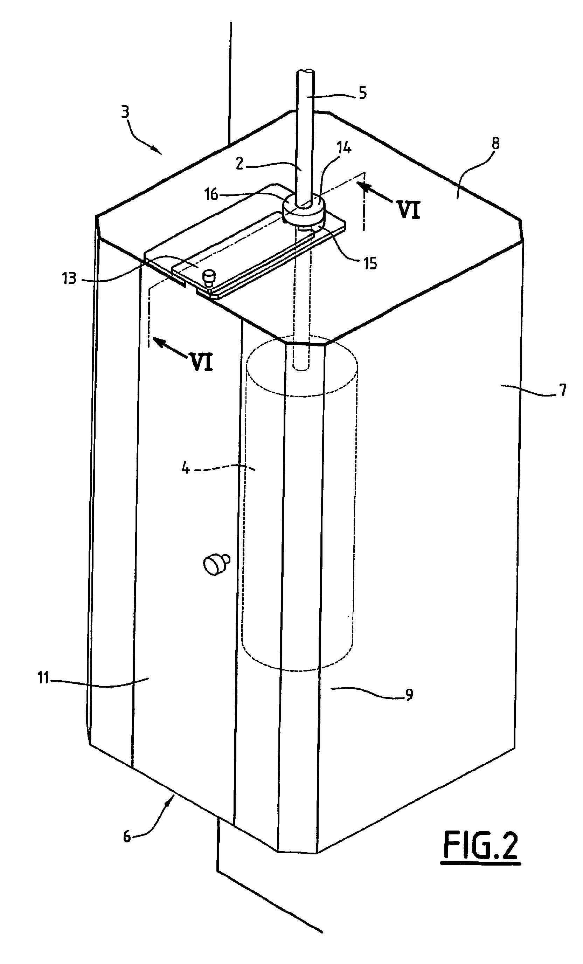

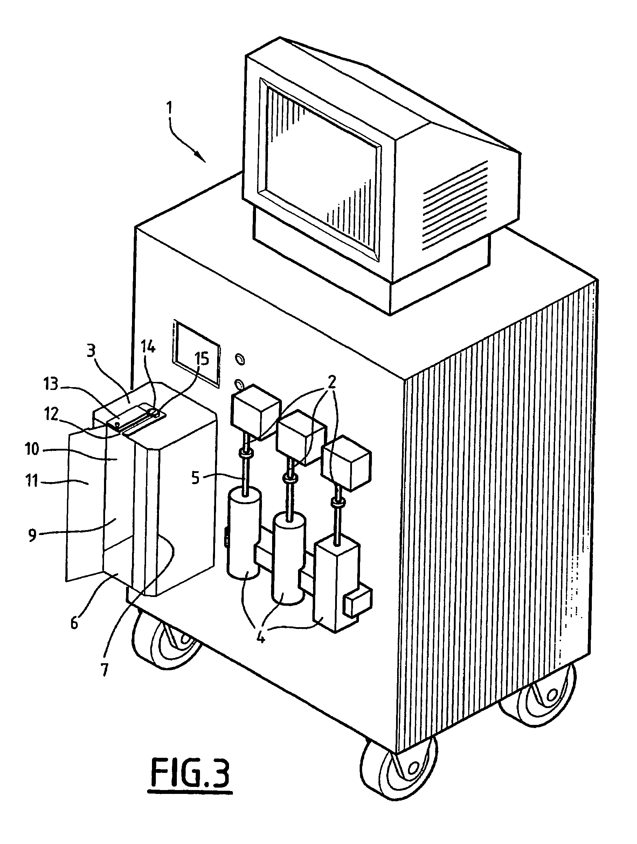

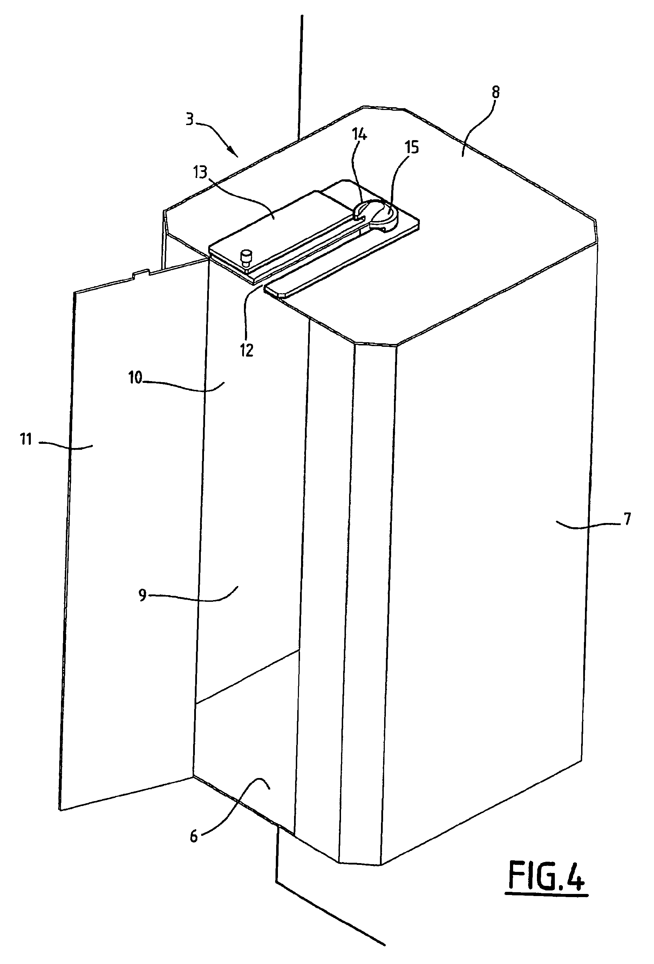

Dans le mode de réalisation représenté sur les figures, la paroi 7 opposée à l'appareil d'imagerie comprend une ouverture 10 qui peut être fermée par une porte 11. La porte est représentée en position fermée sur les Figs. 1 et 2 et en position ouverte sur les Figs. 3 et 4. L'ouverture 10 présente des dimensions adaptées pour le passage de la partie active 4 de la sonde 2. La porte 11 est ouverte pour permettre de disposer la partie active 4 d'une sonde 2 dans le volume de décontamination 9 puis est fermée lors de la désinfection. Lorsque la porte 11 est fermée, le volume 9 est fermé de façon hermétique de sorte à éviter les fuites du principe actif de désinfection vers l'extérieur de l'enceinte 3.

Le capot 8 comprend un passage de câble 12 ouvert vers l'ouverture 10 pour permettre le passage du câble 5 de la sonde 2 lorsque la partie active 4 est introduite dans le volume de décontamination 9. Le passage de câble 12 comporte une fente aménagée dans le capot 8 et s'étendant jusqu'à une partie centrale du capot 8, comme représenté sur les Figs. 3 et 4.

Selon un mode de réalisation représenté sur les figures, le capot 8 comporte en outre un volet d'obturation 13 mobile entre une position de fermeture (Figs. 1 et 2) du passage de câble 12 et une position d'ouverture (Figs. 3 et 4) du passage de câble 12. Le volet 13 ferme le passage du câble 12 de manière hermétique lorsqu'il est en position de fermeture. Ainsi, lorsque la partie active 4 de la sonde 2 a été introduite dans le volume de décontamination 9, la porte 11 et le volet 13 sont mis en position de fermeture de sorte à isoler hermétiquement le volume 9 de l'extérieur de l'enceinte et permettre une désinfection sans risque de la partie active 4. Selon un mode de réalisation, la porte 11 est agencée pour que sa fermeture entraîne le volet mobile 13 vers sa position de fermeture du passage de câble 12. Ainsi, il suffit à l'opérateur de fermer la porte 11 pour que le volet se place automatiquement en position de fermeture, ce qui évite les erreurs de manipulation si l'opérateur oublie d'actionner le volet 13 après avoir disposé la sonde 2 dans l'enceinte 3.

Selon un autre mode de réalisation non représenté, le volet 13 est solidaire de la partie supérieure de la porte 11 et est agencé pour fermer le passage du câble 12 de manière hermétique lorsque la porte est fermée. Un tel mode de réalisation est particulièrement simple à réaliser et ne nécessite pas de moyens de transmission de mouvement entre la porte 11 et le volet 13, contrairement au cas où le volet 13 est porté par le capot 8.

Le capot 8 comprend en outre un organe 14 de suspension de câble au travers du passage de câble 12. L'organe 14 de suspension de câble permet de maintenir le câble 5 de la sonde 2 de sorte que la partie active 4 soit suspendue dans une partie sensiblement centrale du volume de décontamination 9, comme représenté sur les Figs. 1, 2 et 6. On évite ainsi que la partie active 4 touche les parois 7 de l'enceinte 3 et on assure une exposition uniforme de la partie active au principe actif de désinfection.

Selon le mode de réalisation représenté sur les figures, l'organe de suspension 14 comprend une pièce de réception 15 d'une pièce de suspension 16 prévue sur le câble 5 de la sonde 2. La pièce de réception 15 et la pièce de suspension 16 forment un ensemble d'assemblage constitué d'une pièce mâle et d'une pièce femelle. Selon le mode de réalisation représenté sur les figures, la pièce mâle est la pièce de réception 15 et la pièce femelle est la pièce de suspension 16.

Selon un mode de réalisation, l'ensemble d'assemblage est agencé pour que le câble 5 soit orienté d'une façon particulière lorsque la pièce de suspension 16 est fixée sur la pièce de réception 15. Par exemple, l'orientation du câble est obtenue par vissage jusqu'à une position de butée de la pièce de suspension 16 sur la pièce de réception 15.

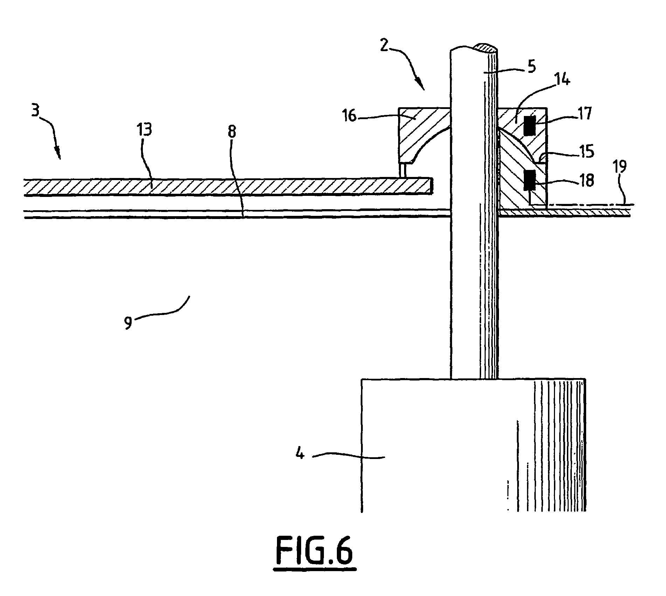

Comme représenté sur la Fig. 6, le volet 13 et l'ensemble d'assemblage sont agencés pour que l'enceinte 3 soit fermée hermétiquement lorsque la pièce de suspension 16 est fixée sur la pièce de réception 15 et que le volet 13 est fermé.

Selon un mode de réalisation non représenté, l'organe de suspension 14 comporte un tube ouvert apte à s'emboîter de manière hermétique sur une portion de câble 5 dont le diamètre est dans une plage de diamètres prédéterminés. Selon divers modes de réalisation, on prévoit des moyens d'orientation du câble 5, par exemple par des moyens d'indexation ou autres, afin que le câble présente une orientation prédéterminée lorsque la partie active 4 est suspendue dans le volume de décontamination 9.

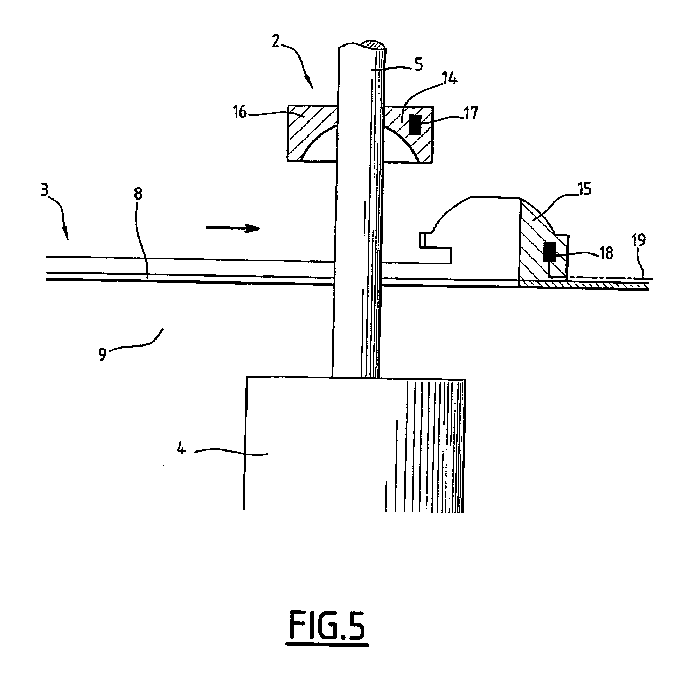

La sonde 2 comprend des moyens d'identification du type étiquette d'identification électronique 17, par exemple une puce RFID. Cette puce contient des informations relatives à l'identité de la sonde. Selon l'invention, l'étiquette 17 est fixée ou noyée dans la pièce de suspension 16, comme représenté sur les Figs. 5 et 6.

L'enceinte comprend des moyens de lecture 18 de l'étiquette d'identification électronique, par exemple du type lecteur RFID. Les moyens de lecture 18 sont agencés pour être à proximité de l'étiquette d'identification 17 lorsque le câble 5 est suspendu à l'organe de suspension 15 de sorte que les moyens de lecture 18 peuvent lire les informations d'identification de l'étiquette d'identification 17. La disposition des moyens de lecture à proximité de l'étiquette d'identification peut être simplifiée par les moyens d'orientation du câble 5. Selon l'invention, les moyens de lecture 18 sont prévus au niveau de l'organe de suspension 15 ou intégrés à celui-ci, comme représenté sur les Figs. 5 et 6. Les moyens de lecture 18 sont par exemple un lecteur RFID de faible portée. Ainsi, le lecteur ne peut lire que les informations contenues dans l'étiquette d'identification fixée au câble 5 de la sonde 2 qui est suspendue dans l'enceinte 2 et non des informations de sondes voisines, telles comme les autres sondes représentées sur la Fig. 1. Les moyens de lecture 18 sont connectés à l'appareil d'imagerie 1 ou à système de traitement de données, par exemple par une connexion filaire 19. Selon une autre réalisation, les moyens de lecture sont connectés par une connexion sans fil.

L'étiquette d'identification 17 de la sonde 2 peut fonctionner de paire avec des moyens de mesure (non représentés) de caractéristiques de la désinfection afin d'assurer une traçabilité de la désinfection de la sonde. On peut par exemple prévoir des moyens de mesure de l'intensité du rayonnement UV et du temps d'exposition de la partie active 4 à ce rayonnement si la désinfection est réalisée par UV. Ces informations peuvent être enregistrées dans l'étiquette d'identification de la sonde ou dans l'appareil d'imagerie 1 avec l'identité de la sonde 2. On obtient ainsi un couplage des informations d'identification de la sonde et des informations de désinfection de cette sonde afin d'assurer une traçabilité de la désinfection.

Selon un mode de réalisation, l'appareil d'imagerie 1 est agencé pour ne pas fonctionner avec une sonde dont la désinfection n'a pas eu lieu ou a été insuffisante.

## Revendications

1. Appareil d'imagerie médical comprenant au moins un instrument médical comprenant une parie active (4) et un câble (5) de connexion à l'appareil médical, l'appareil comprenant en outre une enceinte de décontamination pour instruments médicaux, comportant une base (6), au moins une paroi latérale (7) et un capot supérieur (8) délimitant un volume de décontamination (9), une desdites au moins une paroi latérale (7) comportant une ouverture d'accès (10) au volume de décontamination (9) s'étendant jusqu'au capot supérieur (8), le capot supérieur (8) comportant un passage de câble (12) ouvert vers ladite ouverture et un organe de suspension (14) de câble au travers dudit passage de câble, **caractérisé en ce que** l'organe de suspension (14) comprend une pièce de réception (15) d'une pièce de suspension (16) prévue sur le câble (5), la pièce de réception (15) et la pièce de suspension (16) forment un ensemble d'assemblage constitué d'une pièce mâle et d'une pièce femelle, une étiquette d'identification électronique (17) étant fixée ou noyée dans la pièce de suspension (16) et des moyens de lecture (18) étant prévus au niveau de ou intégrés dans l'organe de suspension (14) de sorte que lesdits moyens de lecture (18) sont agencés pour être à proximité de l'étiquette d'identification (17) lorsque le câble (5) est suspendu à l'organe de suspension (14) de câble.

2. Appareil d'imagerie selon la revendication 1, **caractérisé en ce que** les moyens de lecture (18) d'une étiquette d'identification électronique (17) comprennent un lecteur RFID.

3. Appareil d'imagerie selon la revendication 1 ou 2, **caractérisé en ce que** le passage de câble (12) comporte une fente aménagée dans le capot supérieur (8).

4. Appareil d'imagerie selon l'une quelconque des revendications 1 à 3, **caractérisé en ce que** le capot (8) comporte un volet d'obturation (13) mobile entre une position de fermeture du passage de câble (12), dans laquelle le volet (13) ferme le passage de câble (12) de manière hermétique, et une position d'ouverture du passage de câble (12).

5. Appareil d'imagerie selon la revendication 4, **caractérisée en ce qu'**elle comprend une porte (11) de fermeture de l'ouverture (10) d'accès au volume de décontamination (9), ladite porte étant agencée pour que sa fermeture entraîne le volet mobile (13) vers sa position de fermeture du passage de câble (12).

6. Appareil d'imagerie selon l'une quelconque des revendications 1 à 5, **caractérisé en ce que** l'organe de suspension (14) comprend des moyens d'orientation du câble (5) agencés pour disposer l'étiquette d'identification (17) en regard des moyens de lecture (18) lorsque le câble (5) est suspendu à l'organe de suspension (14) du câble.

7. Appareil d'imagerie selon l'une quelconque des revendications 1 à 6, **caractérisé en ce que** l'organe de suspension (14) de câble comporte un tube ouvert apte à s'emboîter de manière hermétique sur une portion de câble de l'instrument médical dont le diamètre est dans une plage de diamètres prédéterminés.

## Claims

1. Medical imaging apparatus comprising at least one medical instrument comprising an active part (4) and a cable (5) for connection to the medical apparatus, the apparatus additionally comprising a decontamination enclosure for medical instruments, having a base (6), at least one lateral wall (7) and an upper cover (8) delimiting a decontamination space (9), one of said at least one lateral walls (7) having an opening (10) for access to the decontamination space (9) which extends as far as the upper cover (8), the upper cover (8) having a cable through-way (12) open to said opening and a cable suspension element (14) through said cable through-way, **characterised in that** the suspension element (14) comprises a part (15) for receiving a suspension part (16) provided on the cable (5), the receiving part (15) and the suspension part (16) forming an assembly unit composed of a male part and a female part, an electronic identification label (17) being fixed or embedded in the suspension part (16) and reading means (18) being provided on or integrated into the suspension element (14) so that said reading means (18) are arranged to be in the proximity of the identification label (17) when the cable (5) is suspended in the cable suspension element (14).

2. Imaging apparatus according to claim 1, **characterised in that** the means (18) for reading an electronic identification label (17) comprise an RFID reader.

3. Imaging apparatus according to claim 1 or 2, **characterised in that** the cable through-way (12) has a slot provided in the upper cover (8).

4. Imaging apparatus according to any one of claims 1 to 3, **characterised in that** the cover (8) has a closure flap (13) movable between a position shutting off the cable through-way (12), in which position the flap (13) hermetically closes off the cable through-way (12), and a position opening the cable through-way (12).

5. Imaging apparatus according to claim 4, **characterised in that** it comprises a door (11) for closing the opening (10) for access to the decontamination space (9), said door being arranged so that its closure moves the movable flap (13) into its position closing off the cable through-way (12).

6. Imaging apparatus according to any one of claim 1 to 5, **characterised in** the suspension element (14) comprises means for orientation of the cable (5) which are arranged to locate the identification label (17) so that it faces the reading means (18) when the cable (5) is suspended in the cable suspension element (14).

7. Imaging apparatus according to any one of claims 1 to 6, **characterised in that** the cable suspension element (14) has an open tube capable of hermetically fitting over a portion of cable of the medical instrument, the diameter of which is within a range of predetermined diameters.

## Patentansprüche

1. Medizinische Bildgebungsvorrichtung umfassend mindestens ein medizinisches Instrument umfassend mindestens einen wirksamen Teil (4) und ein Verbindungskabel (5) zu der medizinischen Vorrichtung, wobei die Vorrichtung weiterhin ein Dekontaminationsgehäuse für medizinische Instrumente umfasst, umfassend eine Basis (6), mindestens eine Seitenwand (7) und eine Abdeckhaube (8), die einen Dekontaminationsraum (9) begrenzt, wobei die mindestens eine Seitenwand eine Zugangsöffnung (10) zu dem Dekontaminationsraum (9) umfasst, der sich bis zu der Abdeckhaube (8) ausdehnt, wobei die Abdeckhaube (8) einen Kabeldurchgang (12), der in Richtung der Öffnung offen ist und ein Kabel-Aufhängelement (14) quer zu dem Kabeldurchgang umfasst, **dadurch gekennzeichnet, dass** das Aufhängeelement (14) ein Aufnahmestück (15) für ein an dem Kabel (5) vorgesehenes Aufhängestück (16) umfasst, wobei das Aufnahmestück (15) und das Aufhängestück (16) eine Montageeinheit bilden, die aus einem männlichen und einem weiblichen Stück besteht, ein elektronisches Identifikationsetikett (17), das an dem Aufhängestück (16) befestigt oder darin versenkt ist, und Lesemittel (18) an dem Aufhängeelement (14) vorgesehen oder darin eingebaut sind, derart, dass die Lesemittel (18) so ausgebildet sind, dass sie unweit des Identifikationsetiketts (17) vorhanden sind, wenn das Kabel an dem Kabel-Aufhängeelement (14) aufgehängt ist.

2. Medizinische Bildgebungsvorrichtung nach Anspruch 1, **dadurch gekennzeichnet, dass** die Lesemittel (18) von einem elektronischen Identifikationsetikett (17) einen RFID-Leser umfassen.

3. Medizinische Bildgebungsvorrichtung nach Anspruch 1 oder 2, **dadurch gekennzeichnet, dass** der Kabeldurchgang (12) einen Schlitz umfasst, der in der Abdeckhaube (8) ausgebildet ist.

4. Medizinische Bildgebungsvorrichtung nach einem der Ansprüche 1 bis 3, **dadurch gekennzeichnet, dass** die Haube eine Verschlussklappe (13) umfasst, die zwischen Öffnungsposition des Kabeldurchgangs (12), in der die Klappe (13) dien Kabeldurchgang (12) hermetisch verschließt, und einer Öffnungsposition des Kabeldurchgangs (12) beweglich ist.

5. Medizinische Bildgebungsvorrichtung nach Anspruch 4, **dadurch gekennzeichnet, dass** sie eine Verschlusstüre (11) der Öffnung (10) zum Zugang zum Dekontaminationsraum (9) umfasst, wobei die Türe so ausgebildet ist, dass ihr Öffnen die bewegliche Klappe (13) in die Richtung ihrer Verschlussposition des Kabeldurchgangs (12) bewegt.

6. Medizinische Bildgebungsvorrichtung nach einem der Ansprüche 1 bis 5, **dadurch gekennzeichnet, dass** das Aufhängeelement (14) Orientierungsmittel für das Kabel (5) umfasst, die ausgebildet sind, um das Identifikationsetikett (17) neben dem Lesemittel (18) anzuordnen, wenn das Kabel (5) an dem Aufhängeelement (14) des Kabels aufgehängt ist.

7. Medizinische Bildgebungsvorrichtung nach einem der Ansprüche 1 bis 6, **dadurch gekennzeichnet, dass** das Kabel-Aufhängeelement (14) ein offenes Rohr umfasst, das dazu ausgelegt ist, hermetisch auf einen Kabelteil des medizinischen Instruments zu passen, dessen Durchmesser sich in einem Bereich von vorbestimmten Durchmessern befindet.
